# EUROPEAN PATENT SPECIFICATION

(11) **EP 2 913 049 B1**
(45) Date of publication and mention of the grant of the patent: **30.11.2016**
(21) Application number: 15156462.2
(22) Date of filing: 25.02.2015
(51) Int. Cl.: A61K 9/46, A61K 31/192

(54) **Stable pharmaceutical compositions**
Stabile pharmazeutische Zusammensetzungen
Compositions pharmaceutiques stables

(30) Priority: 27.02.2014 EP 14156950
(43) Date of publication of application: 02.09.2015
(73) Proprietor: Galenicum Health S.L., 08005 Barcelona (ES)
(72) Inventor: Arroyo Hidalgo, Sergio, 08005 Barcelona (ES); Puigvert Colomer, Marina, 08005 Barcelona (ES)
(74) Representative: Galenicum Health S.L.

(56) References cited:
- EP-A1- 0 351 353
- EP-A2- 0 228 164
- WO-A2-02/098388

## Description

This invention relates to pharmaceutical compositions of ibuprofen for oral administration. More particularly, the invention relates to pharmaceutical powder compositions containing ibuprofen which effervesce when added to water, forming an aqueous suspension of ibuprofen suitable for oral administration.

### STATE OF THE ART

(*RS*)-2-(4-(2-methylpropyl)phenyl)propanoic acid, also known as ibuprofen, is a well known medicament with anti-inflammatory, antipyretic and analgesic activities. The uses of ibuprofen include the treatment of pain and inflammation in musculoskeletal disorders such as rheumatic disease, and the treatment of pain in a variety of other disorders.

Many ibuprofen based drugs in the form of effervescent powders or tablets have been disclosed. DE3638414 relates to compositions which include basic amino acids to improve the dissolution of ibuprofen. EP0351353 proposes compositions with specific proportions of ibuprofen, sodium bicarbonate and citric acid and a process by granulation in a fluidized bed granulator to obtain completely soluble effervescent tablets of ibuprofen which do not have a bitter taste and do not cause irritation of the throat. EP0877606 discloses effervescent ibuprofen formulations which contain two separate granulates, one containing an acid salt of ibuprofen and a basic salt and prepared by wet granulation, and another containing an effervescent couple. The two granulates are mixed and packed in pouches or pressed into tablets. DE3638414 relates to effervescent tablet compositions which contain ibuprofen lysinate or ibuprofen arginate. EP02023768 relates to fast dissolving ibuprofen effervescent compositions where the ibuprofen containing granulate and the extragranular component have a particular particle size. Finally, EP0228164 relates to compositions where ibuprofen is coated with a polymer and a saccharide is used for stabilizing the composition. EP0228164 discloses effervescent pharmaceutical compositions of ibuprofen where the ibuprofen and the acidic ingredient of the effervescent couple are in a granulate obtained by wet granulation, and the basic ingredient of the effervescent couple is in the extragranular component.

### SUMMARY OF THE INVENTION

This invention relates to pharmaceutical compositions of ibuprofen for oral administration. More particularly, the invention relates to pharmaceutical powder compositions containing ibuprofen, which effervesce when added to water, forming an aqueous suspension of ibuprofen suitable for oral administration. Such compositions are advantageous for those patients, often children and elderly patients, who have difficulty in swallowing tablets or capsules.

The pharmaceutical compositions of ibuprofen in the market very often show effervescence in the sachet or stick pack, which leads to the formation of carbon dioxide and to the inflation of the sachets or stick packs. The pharmaceutical compositions of the present invention are stable over time and do not produce any effervescence in the packaging, even having both of the effervescent couple ingredients in the extragranular component. Said extragranular component is a direct mix of ingredients and does not need to be granulated. Moreover, the compositions of the present invention show a good flowability and a good density, even having a low water content, which results in an easier process for filling up the sachets or stick packs. Also, the compositions of the present invention do not segregate and both the mass uniformity and the content uniformity are very good.

A first aspect of the invention relates to an effervescent pharmaceutical composition comprising a granular component comprising an active agent and an extragranular component comprising a pharmaceutically acceptable effervescent couple comprising an acidic ingredient and a basic ingredient; wherein the active agent is ibuprofen or a pharmaceutically acceptable salt thereof; and wherein the water content of the pharmaceutical composition is between 0.10 % and 3.0 % w/w in respect of the pharmaceutical composition, when measured by Karl Fisher titration.

A second aspect of the invention relates to a sachet or a stick pack comprising the pharmaceutical composition of the first aspect.

A third aspect of the invention relates to a pharmaceutical batch of at least 1,500 units, preferably at least 20,000 units, more preferably at least 50,000 units of the pharmaceutical composition of the first aspect or of the sachet or stick pack of the second aspect.

A fourth aspect of the invention relates to a process for the preparation of an effervescent pharmaceutical composition comprising ibuprofen or a pharmaceutically acceptable salt thereof, comprising the following steps:
i) preparing a granular component comprising ibuprofen or a pharmaceutically acceptable salt thereof;
ii) preparing an extragranular component comprising an effervescent couple comprising an acidic ingredient and a basic ingredient;
iii) mixing the granular component obtained in step (i) and the extragranular component obtained in step (ii); and
iv) optionally packaging the mix obtained in step (iii) in sachets or stick packs.

A fifth aspect of the invention relates to the pharmaceutical composition obtained by the process of the fourth aspect.

A sixth aspect of the invention relates to the pharmaceutical composition of the first or fifth aspect, the sachet or stick pack of the second spect, or the pharmaceutical batch of the third aspect, for use in the treatment of inflammation, fever and/or pain.

A seventh aspect of the invention relates to a cardboard box with a patient information leaflet comprising at least one unit of the pharmaceutical composition of the first or fifth aspect.

### DETAILED DESCRIPTION OF THE INVENTION

In a preferred embodiment of the pharmaceutical composition of the first aspect, the water content of the pharmaceutical composition is between 0.15 % and 2.5 % w/w in respect of the pharmaceutical composition, when measured by Karl Fisher titration. In a more preferred embodiment, the water content of the pharmaceutical composition is between 0.20 % and 2.0 % w/w in respect of the pharmaceutical composition, when measured by Karl Fisher titration. The low water content of the pharmaceutical composition of the present invention avoids the effervescence and consequently the sachets or stick packs maintain their shape because no carbon dioxide is produced before the contact with water for intake. Surprisingly, the compositions with such a low water content have a very good flowability, and show very good content uniformity and very good mass uniformity, because there is no segregation.

The term "pharmaceutically acceptable" indicates that the substance or composition must be compatible chemically and/or toxicologically, with the other ingredients comprising a pharmaceutical composition, and/or the mammal being treated therewith.

The term "effervescent couple" as used herein refers to a pair of pharmaceutically acceptable excipients, one of which is a basic ingredient and the other is an acidic ingredient. The basic ingredient liberates carbon dioxide when it comes in contact with the acidic ingredient and water.

The term "granular component" as used herein refers to the part of the pharmaceutical composition that has been obtained by granulating a powder into larger particles herein called granules or granulate. Said granulation can be either wet granulation or dry granulation.

The term "extragranular component" as used herein refers to the set of ingredients in the pharmaceutical composition which have not been granulated and which do not form part of the granular component.

In a preferred embodiment, the effervescent pharmaceutical composition consists of a granular component comprising an active agent and an extragranular component comprising a pharmaceutically acceptable effervescent couple comprising an acidic ingredient and a basic ingredient; wherein the active agent is ibuprofen or a pharmaceutically acceptable salt thereof; and wherein the water content of the pharmaceutical composition is between 0.10 % and 3.0 % w/w in respect of the pharmaceutical composition, when measured by Karl Fisher titration.

In a preferred embodiment, the active agent is ibuprofen. In a preferred embodiment, the ibuprofen content of the granular component is above 50 % w/w, preferably above 80 % w/w in respect of the total amount of granular component. In a preferred embodiment, the granular component only comprises ibuprofen as active agent. In another preferred embodiment, the granular component only comprises ibuprofen as active agent and the extragranular component does not comprise any active agent. In another preferred embodiment, the granular component only comprises ibuprofen as active agent and the extragranular component comprises at least one active agent.

In a preferred embodiment, the acidic ingredient of the effervescent couple is malic acid or citric acid, preferably malic acid. Preferably, the amount of malic acid is between 28 % and 37 % w/w in respect of the total amount of the pharmaceutical composition.

In a preferred embodiment, the pharmaceutical composition of the present invention comprises a surfactant in the extragranular component. Preferably, it comprises an ionic surfactant. More preferably, said surfactant is sodium lauryl sulfate. In a preferred embodiment, the extragranular phase comprises sodium lauryl sulfate in an amount between 0.5 % and 2 % w/w, preferably between 0.6 % and 1.5 % w/w, more preferably between 0.7 % and 1 % w/w, in respect of the total amount of the pharmaceutical composition. The best results of flowability are obtained when the extragranular component comprises a surfactant, preferably sodium lauryl sulfate, especially when the amount of sodium lauryl sulfate is between 0.7 % and 1 % w/w, in respect of the total amount of the pharmaceutical composition.

The term "surfactant" as used herein refers to a pharmaceutically acceptable excipient that reduces the surface tension between two liquids or between a solid and a liquid, facilitating the moistening and therefore increasing the solubility. Anionic surfactants contain anionic functional groups, such as sulfate, sulfonate, phosphate, and carboxylates.

In a preferred embodiment, the basic ingredient of the effervescent couple is selected from sodium hydrogen carbonate, sodium carbonate anhydrous and a mixture thereof, preferably is a mixture thereof.

In a preferred embodiment, the granular component further comprises microcrystalline cellulose, croscarmellose sodium and colloidal silicon dioxide. Preferably, the granular component is obtained by dry granulation, preferably by roller-compactor.

In a preferred embodiment, the extragranular component comprises malic acid, sodium saccharin, sodium lauryl sulfate, sucrose, sodium hydrogen carbonate, sodium carbonate anhydrous and a flavouring agent.

In a preferred embodiment, the pharmaceutical composition comprises 200 mg or 400 mg or 600 mg of ibuprofen per unit dose, preferably 600 mg per unit dose.

As used herein, the term "unit dose" or "unit dosage" refers to a physically discrete unit that contains a predetermined quantity of active ingredient calculated to produce a desired therapeutic effect. The unit dose or unit dosage may be in the form of a tablet, capsule, sachet, etc. referred to herein as a "unit dosage form."

In a preferred embodiment of the second aspect, the sachet or stick pack consists essentially of a paper foil/polyethylene foil/aluminium foil/polyethylene foil laminate or of a polyethylene terephthalate foil/polyethylene foil/aluminium foil/polyethylene foil laminate.

In a preferred embodiment of pharmaceutical batch of the third aspect, the relative standard deviation value of the mass uniformity is less than 7.5 % w/w, preferably less than 5 % w/w, more preferably less than 2 % w/w.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active ingredient in the pharmaceutical composition units (e.g. tablets, capsules, sachets, stick packs, etc) of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The term "active ingredient" refers to a therapeutically active compound, as well as any prodrugs thereof and pharmaceutically acceptable salts, hydrates and solvates of the compound and the prodrugs.

For the release of a pharmaceutical batch the distribution of the active ingredient in the composition has to be homogeneous, that is, content uniformity is required.

All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is essential, therefore, to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

In a preferred embodiment of the process of the fourth aspect, wherein the granular component is prepared by dry granulation, more preferably by roller-compactor. The granular component can be prepared by wet or dry granulation. Preferably, it is prepared by dry granulation. More preferably, the dry granulation is done using a roller compactor.

In another preferred embodiment, the extragranular component is prepared by direct mix. The pharmaceutical compositions as disclosed herein can be compressed into effervescent tablets, can be filled into capsules or can be packaged in sachets or stick packs. The tablets or capsules can then be packaged in bottles or blisters. In a preferred embodiment, the pharmaceutical compositions as disclosed herein are packaged in sachets or stick packs.

The term "sachet" or "stick pack" as used herein refers to a small, sealed packet containing a quantity of material, which is a single use or unit dose quantity. A packaging of the stick pack type, preferably with improved opening, comprises normally: a flexible film, having at least one layer, which forms a hermetically sealed tubular body with mutually opposite longitudinal film flaps, a first band provided longitudinally to said body for inside/outside sealing of said mutually opposite longitudinal flaps of the film; second sealing bands provided transversely to said body for inside/outside sealing; a sealed extension region protruding from at least one of said second sealing bands on a respective portion of at least one edge of said tubular body; and preferably a transverse pre-weakening incisions that are provided in longitudinal alignment with said sealed extension region, along at least one of said mutually opposite longitudinal flaps. Suitable stick packs are described in WO9501921.

In a preferred embodiment of the process of the fourth aspect, the process is performed at less than 25 % relative humidity and less than 22 °C.

In a preferred embodiment of the seventh aspect, the pharmaceutical composition is packaged in a sachet or stick pack. In a preferred embodiment, the cardboard box comprises at least one sachet or at least one stick pack comprising the effervescent pharmaceutical composition of the present invention.

The term "blister" or bubble pack refers to a sheet in a package construction with recesses designed to hold dosage forms. The sheet may be a plastic, a foil, or combination thereof. Normally, a blister is a product consisting of a flat structure in which blisters are formed, generally by means of a heating process, into which the single elements to be packaged are inserted. The blisters are then hermetically sealed using flat strips of appropriate thermomoldable materials (plastics, aluminum, paper), which represent the frangible element through which it is then possible to remove the product. The primary component of a blister pack is a cavity or pocket made from a formable web, usually a thermoformed plastic. This usually has a backing of paperboard or a lidding seal of aluminum foil or plastic. An aluminium/PVC blister refers to a blister the thermomoldable material is from PVC and the backing is a lidding seal of aluminium foil. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and re-packaging are not common. A series of blister cavities is sometimes called a blister card or blister strip as well as blister pack. The difference between a strip pack and blister pack is that a strip pack does not have thermo-formed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsable to be able to dispense the tablet or capsule by means of pressing it out with your thumb. The main advantages of unit-dose blister packs over other methods of packing pharmaceutical products are the assurance of product/packaging integrity (including shelf life) of each individual dose and the possibility to create a compliance pack or calendar pack by printing the days of the week above each dose. Blister packs can be created by means of a form-fill-seal process at the pharmaceutical company or designated contract packer. A form-fill-seal process means that the blister pack is created from rolls of flat sheet or film, filled with the pharmaceutical product and closed (sealed) on the same equipment. Such equipment is called a blister line. There are two types of blister machine's design: rotary and flat-plate.

Unless otherwise indicated, all the analysis methods are carried out according to the European Pharmacopoeia 7th edition.

### DESCRIPTION OF THE FIGURES

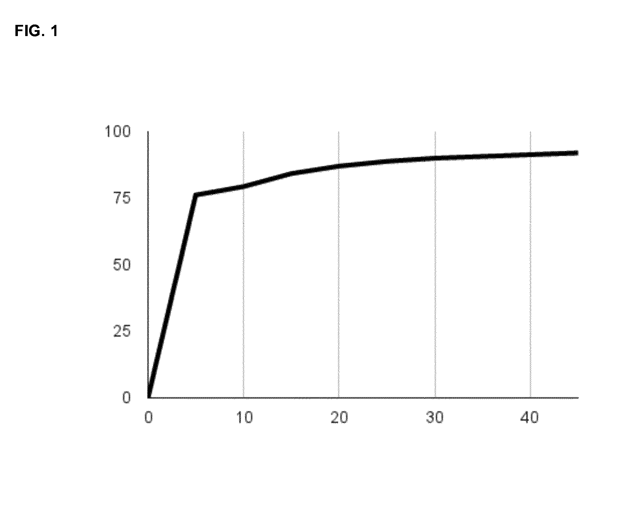

Figure 1 depicts the dissolution profile of the composition of example 1a, tested in a paddle apparatus at 50 rpm and with pH 6.8 phosphate buffer as dissolution medium. The figure represents the percent of ibuprofen dissolved vs time in minutes.

### EXAMPLES

The following examples illustrate various embodiments of the invention and are not intended to limit the invention in any way.

### Example 1. Compositions.

| | Ex. 1 a (mg) | Ex. 1 b (mg) |
|---|---|---|
| Ibuprofen granulate (DC 85 BASF) | 706* | 706* |
| DL Malic acid | 1190 | 906 |
| Sodium saccharin | 27 | 27 |
| Sodium lauryl sulfate | 28 | 28 |
| Orange flavour | 60 | 60 |
| Sucrose | 904 | 904 |
| Sodium hydrogen carbonate | 400 | 304 |
| Sodium carbonate anhydrous | 85 | 65 |

| | | |
|---|---|---|
| * containing 600 mg of ibuprofen. | | |

The ibuprofen granulate used was Ibuprofen DC 85 BASF, which comprise 85 % w/w ibuprofen, 6.6 % w/w microcrystalline cellulose, 5.4 % w/w colloidal silicon dioxide and 2.9 % w/w croscarmellose sodium in respect of the total amount of the granules and are prepared by roller compactor dry granulation (WO2007042445; US20130078287).

### Example 2. Manufacturing process.

Batches of at least 1,500 sachets or stick packs of the compositions of example 1 were manufactured as follows: first, the granulate comprising the ibuprofen was sieved consecutively using a 1.25 mm, then a 1.0 mm and then a 0.8 mm sieve. The malic acid, sodium saccharin, sodium lauryl sulfate, orange flavour and sucrose were weighed, sieved and mixed with the ibuprofen granules in a suitable blender for 15 minutes. The sodium hydrogen carbonate and the sodium carbonate anhydrous were weighed and sieved, and they were added to the previous blend and mixed for 20 minutes. This final mix can be stored in an air-tight container before packaging in sachets or stick packs of paper/polythene/aluminium foil/polythene laminate or of polyethylene terephthalate foil/polyethylene foil/aluminium foil/polyethylene foil laminate. The whole process was performed under less than 25 % relative humidity and less than 22 °C.

### Example 3. Water content

The water content of the granular component, the extragranular component and of the complete mix of the pharmaceutical compositions of example 1, manufactured as indicated in example 2, was calculated by Karl Fisher titration.

| Water content (%) | Composition of ex. 1a | Composition of ex. 1b |
|---|---|---|
| Ibuprofen granulate | 0.30 | 0.30 |
| Extragranular component | 0.38 | 0.40 |
| Total composition | 0.37 | 0.38 |

### Example 4. Dissolution profile.

The compositions of the present invention have an immediate release dissolution profile. The dissolution profile was tested in a paddle apparatus at 50 rpm and with pH 6.8 phosphate buffer as dissolution medium. In all the compositions of the examples, more than 80 % of the ibuprofen was dissolved in 45 minutes.

### Example 5. Flowability.

The compositions of the present invention have very good flowability. The Hausner ratio and the flow rate of the compositions of the examples was tested in order to check their flowability. The Hausner ratio is determined by measuring both the bulk volume and tapped volume of a powder. This parameter is considered a simple, fast, and popular method of evaluating powder flow characteristics.

The Hausner ratio of the pharmaceutical compositions of examples 1a and 1b, manufactured as explained in example 2, was analysed as described in the European Pharmacopoeia. The composition of example 1a had a Hausner ratio of 1.14 and the composition of example 1 b had a Hausner ratio of 1.13.

The flowability of the pharmaceutical compositions of examples 1a and 1b, manufactured as explained in example 2, was analysed as described in the European Pharmacopoeia (diameter 10 mm). The composition of example 1a had a flowability of 12.3 g/s and the composition of example 1b had a flowability of 12.0 g/s.

## Claims

1. An effervescent pharmaceutical composition comprising a granular component comprising an active agent and an extragranular component comprising a pharmaceutically acceptable effervescent couple comprising an acidic ingredient and a basic ingredient; wherein the active agent is ibuprofen or a pharmaceutically acceptable salt thereof; and wherein the water content of the pharmaceutical composition is between 0.10 % and 3.0 % w/w in respect of the pharmaceutical composition, when measured by Karl Fisher titration.

2. The pharmaceutical composition according to the preceding claim, wherein the water content of the pharmaceutical composition is between 0.15 % and 2.5 % w/w, preferably between 0.20 % and 2.0 % w/w in respect of the pharmaceutical composition, when measured by Karl Fisher titration.

3. The pharmaceutical composition according to any one of the preceding claims, wherein the ibuprofen content of the granular component is above 50 % w/w, preferably above 80 % w/w in respect of the total amount of granular component.

4. The pharmaceutical composition according to any one of the preceding claims, wherein the acidic ingredient of the effervescent couple is malic acid or citric acid, preferably malic acid, and/or wherein the amount of malic acid is between 28 % and 37 % w/w in respect of the total amount of the pharmaceutical composition.

5. The pharmaceutical composition according to any one of the preceding claims, wherein the extragranular phase comprises sodium lauryl sulfate in an amount between 0.5 % and 2 % w/w, preferably between 0.6 % and 1.5 % w/w, more preferably between 0.7 % and 1 % w/w, in respect of the total amount of the pharmaceutical composition.

6. The pharmaceutical composition according to any one of the preceding claims, wherein the basic ingredient of the effervescent couple is selected from sodium hydrogen carbonate, sodium carbonate anhydrous and a mixture thereof, preferably is a mixture thereof.

7. The pharmaceutical composition according to any one of the preceding claims, wherein the extragranular component comprises malic acid, sodium saccharin, sodium lauryl sulfate, sucrose, sodium hydrogen carbonate, sodium carbonate anhydrous and a flavouring agent.

8. The pharmaceutical composition according to any one of the preceding claims, wherein the composition comprises 200 mg or 400 mg or 600 mg of ibuprofen per unit dose, preferably 600 mg per unit dose.

9. A sachet or a stick pack comprising the pharmaceutical composition according to any one of the preceding claims, preferably wherein said sachet or stick pack consists essentially of a paper foil/polyethylene foil/aluminium foil/polyethylene foil laminate or of a polyethylene terephthalate foil/polyethylene foil/aluminium foil/polyethylene foil laminate.

10. A pharmaceutical batch of at least 1,500 units, preferably at least 20,000 units, more preferably at least 50,000 units of the pharmaceutical composition as defined in any one of claims 1 to 8 or of the sachet or stick pack as defined in claim 9.

11. The pharmaceutical batch according to the preceding claim, wherein the relative standard deviation value of the mass uniformity is less than 7.5 % w/w, preferably less than 5 % w/w, more preferably less than 2 % w/w.

12. A process for the preparation of an effervescent pharmaceutical composition comprising ibuprofen or a pharmaceutically acceptable salt thereof, comprising the following steps:
i) preparing a granular component comprising ibuprofen or a pharmaceutically acceptable salt thereof, preferably by dry granulation, more preferably by roller-compactor;
ii) preparing an extragranular component comprising an effervescent couple comprising an acidic ingredient and a basic ingredient, preferably by direct mix;
iii) mixing the granular component obtained in step (i) and the extragranular component obtained in step (ii); and
iv) optionally packaging the mix obtained in step (iii) in sachets or stick packs.

13. The pharmaceutical composition obtained by the process of claim 12.

14. The pharmaceutical composition according to any one of claims 1 to 8 or 13, the sachet or stick pack as defined in claim 9, or the pharmaceutical batch according to any one of claims 10 or 11, for use in the treatment of inflammation, fever and/or pain.

15. A cardboard box with a patient information leaflet comprising at least one unit of the pharmaceutical composition as defined in any one of claims 1 to 8 or 13, preferably wherein the pharmaceutical composition is packaged in a sachet or stick pack.

## Patentansprüche

1. Eine sprudelnde pharmazeutische Zusammensetzung aus einem granularen Element, das aus einem Wirkstoff besteht, der ein extragranulares Element, ein pharmazeutisch anerkanntes Paar säurehaltiger und basischer Inhaltsstoffe, umfasst; wobei Ibuprofen oder ein pharmazeutisches anerkanntes Salz daraus den Wirkstoff darstellt; und wobei der Wassergehalt der pharmazeutischen Zusammensetzung zwischen 0,10 % und 3,0 % w/w hinsichtlich der pharmazeutischen Zusammensetzung, wenn nach der Karl Fisher Titration gemessen, liegt.

2. Die pharmazeutische Zusammensetzung gemäß dem vorhergehenden Anspruch, wobei der Wassergehalt der pharmazeutischen Zusammensetzung zwischen 0,15 % und 2,5 % w/w, vorzugsweise zwischen 0,20 % und 2,0 % w/w hinsichtlich der pharmazeutische Zusammensetzung liegt, wenn nach der Karl Fisher Titration gemessen.

3. Die pharmazeutische Zusammensetzung gemäß einem der vorhergehenden Ansprüche, wobei der Ibuprofengehalt des extragranularen Elements über 50 % w/w, vorzugsweise über 80 % w/w hinsichtlich der Gesamtmenge des körnigen Bestandteils liegt.

4. Die pharmazeutische Zusammensetzung gemäß einem der vorhergehenden Ansprüche, bei der der säurehaltige Inhaltsstoff der Brausekombination Apfel- oder Zitronensäure darstellt, vorzugsweise Apfelsäure, und/oder bei der der Anteil der Apfelsäure zwischen 28 % und 37 % w/w hinsichtlich der Gesamtmenge der pharmazeutische Zusammensetzung liegt.

5. Die pharmazeutische Zusammensetzung gemäß einem der vorhergehenden Ansprüche, bei der die extragranulare Phase Natriumlaurylsulfat in einer Menge zwischen 0,5 % und 2 % w/w, vorzugsweise zwischen 0,6 % und 1,5 % w/w, hinsichtlich der Gesamtmenge der pharmazeutischen Zusammensetzung umfasst.

6. Die pharmazeutische Zusammensetzung gemäß einem der vorhergehenden Ansprüche, bei der der basische Inhaltsstoff der Brausekombination aus Natriumhydrogenkarbonat, wasserfreiem Natriumkarbonat und einer Mischung daraus, vorzugsweise aber einer Mischung daraus, ausgewählt wird.

7. Die pharmazeutische Zusammensetzung gemäß einem der vorhergehenden Ansprüche, bei der das extragranulare Element aus Apfelsäure, Natriumsachharin, Natriumlaurylsulfat, Sucrose, Natriumhydrogencarbonat, wasserfreiem Natriumkarbonat und einem Aromastoffs besteht.

8. Die pharmazeutische Zusammensetzung gemäß einem der vorhergehenden Ansprüche, bei der die Zusammensetzung aus 200 mg oder 400 mg oder 600 mg des Ibuprofens pro Dosierungseinheit vorzugsweise aus 600 mg pro Dosierungseinheit besteht.

9. Ein Beutel oder Stickpack enthält die pharmazeutische Zusammensetzung gemäß einem der vorhergehenden Ansprüchen, wobei der Beutel oder Stick Pack vorzugsweise im Wesentlichen aus Papierfolie/Polyethylenfolie/Aluminiumfolie/Polyethylenfolienlaminat oder aus PET-Folie/Polyethylenfolie/Aluminiumfolie/Polyethylenfolienlaminat besteht.

10. Eine pharmazeutische Charge von mindestens 1 500 Einheiten, vorzugsweise von mindestens 20 000 Einheiten, noch bevorzugter sogar von mindestens 50 000 Einheiten der wie in den Ansprüchen 1 bis 8 bestimmt pharmazeutischen Zusammensetzung oder des in Anspruch 9 definierten Beutel und Stick Packs.

11. Die pharmazeutische Charge gemäß dem vorhergehenden Anspruch, wobei der verhältnismäßige Standardabweichungswert der Masseneinheit weniger als 7,5 % w/w, vorzugsweise weniger als 5 % w/w, noch vorzugswürdiger weniger als 2% w/w beträgt.

12. Ein Prozess für die Vorbereitung der sprudelnden pharmazeutischen Zusammensetzung, die Ibuprofen oder ein pharmazeutisch anerkanntes Salz daraus enthält, der die folgenden Schritte beinhaltet:
i) Ein extragranulares Element vorzubereiten das Ibuprofen oder ein pharmazeutisch anerkanntes Salz daraus beinhaltet, bevorzugt durch trockene Granulierung, noch bevorzugter durch Walzenpresse;
ii) Ein extragranulares Element vorzubereiten das ein Brausekombination, welche einen säurehaltigen Inhaltsstoff und einen basischen Inhaltsstoff enthält, vorzugsweise direkt durch Mischung;
iii) Das granulare Element, welches in Schritt i) gewonnen wurde, mit dem in Schritt ii) gewonnenen extragranularen Element zu mischen; und
iv) Die in Schritt iii) hergestellte Mischung optional in Beutel oder Stick Packs zu verpacken.

13. Die pharmazeutische Zusammensetzung, die während des in Anspruch 12 beschriebenen Prozesses gewonnen wird.

14. Die pharmazeutische Zusammensetzung gemäß einem der Ansprüche 1 bis 8 oder 13, der Beutel oder Stick Pack wie in Anspruch 9 definiert, oder die pharmazeutische Charge im Sinne der Ansprüche 10 oder 11, für den Gebrauch im Rahmen der Behandlung von Entzündungen, Fieber und/oder Schmerzen.

15. Eine Pappkartonkiste mit einem Patienteninformationsblatt, die zumindest eine Einheit der in den Ansprüchen 1 bis 8 oder 13 definierten pharmazeutischen Zusammensetzung enthält, wobei die pharmazeutische Zusammensetzung vorzugsweise in einem Beutel oder Stick Pack verpackt ist.

## Revendications

1. Composition pharmaceutique effervescente comprenant un composant granulaire comprenant un agent actif et un composant extragranulaire comprenant un couple effervescent pharmaceutiquement acceptable comprenant un ingrédient acide et un ingrédient basique; dans laquelle le principe actif est l'ibuprofène ou un sel pharmaceutiquement acceptable de celui-ci; et dans laquelle la teneur en eau de la composition pharmaceutique est entre 0,10% et 3,0% p/p par rapport à la composition pharmaceutique, mesurée par titrage Karl Fisher.

2. Composition pharmaceutique selon la revendication précédente, dans laquelle la teneur en eau de la composition pharmaceutique est entre 0,15% et 2,5% p/p, de préférence entre 0,20% et 2,0% p/p par rapport à la composition pharmaceutique, mesurée par titrage Karl Fisher.

3. Composition pharmaceutique selon l'une quelconque des revendications précédentes, dans laquelle la teneur en ibuprofène du composant granulaire est supérieure à 50% p/p, de préférence supérieure à 80% p/p par rapport à la quantité totale du composant granulaire.

4. Composition pharmaceutique selon l'une quelconque des revendications précédentes, dans laquelle l'ingrédient acide du couple effervescent est l'acide malique ou l'acide citrique, de préférence l'acide malique et/ou dans laquelle la quantité d'acide malique est entre 28% et 37% p/p par rapport à la quantité totale de la composition pharmaceutique.

5. Composition pharmaceutique selon l'une quelconque des revendications précédentes, dans laquelle la phase extragranulaire comprend le laurylsulfate de sodium en une quantité entre 0,5% et 2% p/p, de préférence entre 0,6% et 1,5% p/p, plus préférablement entre 0,7% et 1% p/p, par rapport à la quantité totale de la composition pharmaceutique.

6. Composition pharmaceutique selon l'une quelconque des revendications précédentes, dans laquelle l'ingrédient basique du couple effervescent est choisi parmi le bicarbonate de sodium, le carbonate de sodium anhydre et un mélange de ceux-ci, de préférence un mélange de ceux-ci.

7. Composition pharmaceutique selon l'une quelconque des revendications précédentes, dans laquelle le composant extragranulaire comprend l'acide malique, la saccharine sodique, le laurylsulfate de sodium, le saccharose, le bicarbonate de sodium, le carbonate de sodium anhydre et un agent aromatisant.

8. Composition pharmaceutique selon l'une quelconque des revendications précédentes, dans laquelle la composition comprend 200 mg ou 400 mg ou 600 mg d'ibuprofène par dose unitaire, de préférence 600 mg par dose unitaire.

9. Un sachet ou un stick pack comprenant la composition pharmaceutique selon l'une quelconque des revendications précédentes, de préférence dans lequel ledit sachet ou stick pack se compose essentiellement de feuille de papier/feuille de polyéthylène/feuille d'aluminium/feuille de polyéthylène stratifié ou de feuille de polyéthylène téréphtalate/feuille de polyéthylène/feuille d'aluminium/feuille de polyéthylène stratifié.

10. Un lot pharmaceutique d'au moins 1.500 unités, de préférence au moins 20.000 unités, de préférence au moins 50.000 unités de la composition pharmaceutique telle que définie dans l'une quelconque des revendications 1 à 8 ou du sachet ou du stick pack tel que défini dans la revendication 9.

11. Le lot pharmaceutique selon la revendication précédente, dans lequel la valeur du coefficient de variation de l'uniformité de masse est inférieure à 7,5% p/p, de préférence inférieure à 5% p/p, plus préférablement inférieure à 2% p/p.

12. Procédé pour la préparation d'une composition pharmaceutique comprenant de l'ibuprofène effervescent ou d'un sel pharmaceutiquement acceptable de celui-ci, comprenant les étapes suivantes:
i) préparation d'un composant granulaire comprenant de l'ibuprofène ou un sel pharmaceutiquement acceptable de celui-ci, de préférence par granulation en voie sèche, de préférence dans un compacteur à rouleaux;
ii) la préparation d'un composant extragranulaire comprenant un couple effervescent comprenant un ingrédient acide et un ingrédient basique, de préférence par mélange directe;
iii) mélanger le composant granulaire obtenu dans l'étape (i) et le composant extragranulaire obtenu à l'étape (ii); et
iv) éventuellement le conditionnement du mélange obtenu à l'étape (iii) dans des sachets ou des stick packs.

13. La composition pharmaceutique obtenue par le procédé de la revendication 12.

14. Composition pharmaceutique selon l'une quelconque des revendications 1 à 8 ou 13, le sachet ou le stick pack tel que défini dans la revendication 9 ou le lot pharmaceutique selon l'une quelconque des revendications 10 ou 11, en vue d'une utilisation dans le traitement de l'inflammation, de la fièvre et/ou de la douleur.

15. Une boîte en carton avec une notice patient, comprenant au moins une unité de la composition pharmaceutique telle que définie dans l'une quelconque des revendications 1 à 8 ou 13, de préférence dans laquelle la composition pharmaceutique est conditionnée dans un emballage en sachet ou stick pack.
